# EUROPÄISCHE PATENTANMELDUNG

(11) **EP 3 018 196 A1**
(43) Veröffentlichungstag der Anmeldung: **11.05.2016**
(21) Anmeldenummer: 15192780.3
(22) Anmeldetag: 03.11.2015
(51) Int. Cl.: C12M 3/00, C12M 1/00, C12M 1/42, C12M 1/34, B01L 3/00, C12M 1/30, C12Q 1/04, C12Q 1/68, G01N 21/00

(54) **VORRICHTUNG UND VERFAHREN ZUR IDENTIFIZIERUNG VON MIKROORGANISMEN**

(30) Priorität: 04.11.2014 DE 102014116050
(71) Anmelder: Universität Potsdam, 14469 Potsdam (DE)
(72) Erfinder: Niedl, Robert, 14089 Berlin (DE); Beta, Carsten, 12163 Berlin (DE)
(74) Vertreter: Müller & Schubert

(57) **Zusammenfassung**

Die vorliegende Erfindung betrifft eine Vorrichtung zur Identifizierung von Mikroorganismen umfassend:
a) einen Träger 1, wobei auf dem Träger 1 ein saugfähiges Material 2 angeordnet ist,
b) mindestens eine abdichtende Zwischenschicht 3, welche mindestens eine Aussparung 4 aufweist, welche zur Aufnahme zu identifizierender Mikroorganismen, die auf einem Mikroporenfilter 5 vorhanden sind, ausgestaltet ist,
c) mindestens ein Reaktionsfließ 6 bestehend aus einem saugfähigen Material 2, auf dem mindestens ein Absorptionsfeld 7 und/oder mindestens eine flüssigkeitführende Struktur 8 zur Aufnahme von Reagenzien zur Identifizierung der Mikroorganismen angeordnet ist, und
d) eine Deckplatte 9.

Weiterhin betrifft die vorliegende Erfindung ein Verfahren zur Identifizierung von Mikroorganismen unter Verwendung der erfindungsgemäßen Vorrichtung.

Durch die vorliegende Erfindung können Mikroorganismen in einer Probe sehr schnell, innerhalb von wenigen Minuten, identifiziert werden. Dabei ist das Testsystem von der Umwelt isoliert, wodurch eine Gefährdung von Mensch und Umwelt bei einem positiven Testbefund vermieden wird.

## Beschreibung

Gegenstand der vorliegenden Erfindung ist eine Vorrichtung und ein Verfahren zur Identifizierung von Mikroorganismen.

Mit Änderung der Trinkwasserverordnung (TrinkwV2001) wurde festgelegt, dass Warmwasserspeichersysteme mit einem Speichervolumen von mehr als 200 Litern und Trinkwarmwasserleitungssysteme mit einem Fassungsvolumen von mehr als 3 Litern regelmäßig auf Mikroorganismen wie *Legionellen* oder *E. coli.* Bakterien untersucht werden müssen. Darüber hinaus müssen Beatmungssysteme wie mobile Beatmungsautomaten, Sauerstoff- oder Beatmungsluftleitungen in Krankenhäusern etc. regelmäßig kontrolliert und gewartet werden.

Bisher werden diese Tests über labordiagnostische Analysen von vorher entnommenen Probenlösungen oder Filtervorrichtungen durchgeführt. Hierbei werden spezielle mikrobiologische Nährböden mit Testausstrichen versehen und nach entsprechenden Bebrütungszeiten bei optimalen Bedingungen auf die Zielorganismen hin untersucht. Diese beschriebenen Testmethoden nehmen bislang mehrere Tage bis Wochen in Anspruch, so dass entsprechende Anlagen mit Befund noch in der Testzeit in Betrieb sind, oder vorher positiv getestete Anlagen für den Analysezeitraum noch nicht freigegeben sind, obwohl keine Keimbelastung mehr vorliegt.

Aufgabe der vorliegenden Erfindung ist es daher, die Nachteile des Standes der Technik zu überwinden.

Die Aufgabe der Erfindung ist es eine Vorrichtung zur Identifizierung von Mikroorganismen bereitzustellen. Mittels dieser Vorrichtung soll die Identifizierung der Mikroorganismen in wenigen Minuten erfolgen. Dabei soll die Sensitivität mindestens gleich zu der im Stand der Technik bekannten Systemen sein und die gesetzlich akzeptierten Analysemethoden einhalten.

Des Weiteren ist es eine Aufgabe der Erfindung eine Vorrichtung bereitzustellen, die von der Umwelt isoliert ist, um eine Gefährdung von Menschen und Umwelt bei einem positiven Befund zu vermeiden.

Außerdem ist es eine Aufgabe der Erfindung eine Vorrichtung bereitzustellen, die eine Einmalanwendung ermöglicht.

Die Aufgabe der vorliegenden Erfindung wird durch die Bereitstellung einer Vorrichtung zur Identifizierung von Mikroorganismen gelöst, welche die Merkmale des Hauptanspruchs aufweist. Vorteilhafte Weiterbildungen der erfindungsgemäßen Vorrichtung sind in den abhängigen Unteransprüchen dargestellt.

Gegenstand der Erfindung ist eine Vorrichtung zur Identifizierung von Mikroorganismen umfassend:
a) einen Träger, wobei auf dem Träger ein saugfähiges Material angeordnet ist,
b) mindestens eine abdichtende Zwischenschicht, welche mindestens eine Aussparung aufweist, welche zur Aufnahme zu identifizierender Mikroorganismen, die auf einem Mikroporenfilter vorhanden sind, ausgestaltet ist,
c) mindestens ein Reaktionsfließ bestehend aus einem saugfähigen Material, auf dem mindestens ein Absorptionsfeld und/oder mindestens eine flüssigkeitführende Struktur zur Aufnahme von Reagenzien zur Identifizierung der Mikroorganismen angeordnet ist, und
d) eine Deckplatte.

Erfindungsgemäß bevorzugt ist eine Vorrichtung, bei der der Träger und/oder die Deckplatte aus Glas, Polyethylen, Polypropylen, Polyvinylchlorid, Polystyrol, Cyclo-Olefin-Copolymere, Polymethyl-methacrylat oder Polyurethan besteht.

Besonders bevorzugt ist eine erfindungsgemäße Vorrichtung, bei der das saugfähige Material aus Papier, cellulosebasierenden Substraten, Polyethylen, Polyethersulfon oder Polycarbonat besteht.

Insbesondere bevorzugt ist eine erfindungsgemäße Vorrichtung, bei der die abdichtende Zwischenschicht aus einem flüssigkeitsundurchlässigen Material besteht.

Außerdem ist eine erfindungsgemäße Vorrichtung bevorzugt, bei der die abdichtende Zwischenschicht fest mit dem saugfähigen Material verklebt oder verpresst ist.

Weiterhin ist eine erfindungsgemäße Vorrichtung bevorzugt, bei der mehr als ein Reaktionsfließ und mehr als eine abdichtende Zwischenschicht vorgesehen ist, wobei das Reaktionsfließ und die abdichtende Zwischenschicht abwechselnd angeordnet sind.

Bevorzugt ist insbesondere eine erfindungsgemäße Vorrichtung, bei der auf dem mindestens einen Absorptionsfeld mindestens ein schaltbares Speicherreservoir angeordnet ist.

Bevorzugt ist eine erfindungsgemäße Vorrichtung, bei der das mindestens eine Speicherreservoir mit einer flüssigkeitführenden Struktur in Verbindung steht.

Besonders bevorzugt ist eine erfindungsgemäße Vorrichtung, bei der das schaltbare Speicherreservoir ein Polymergel ist.

Insbesondere bevorzugt ist eine Vorrichtung, bei der innerhalb des Speicherreservoirs ein Lösemittel und/oder Reagenz eingebracht ist.

Weiterhin bevorzugt ist eine erfindungsgemäße Vorrichtung, bei der innerhalb des mindestens einen Absorptionsfeldes und/oder der mindestens einen flüssigkeitführenden Struktur mindestens eine Aufnahmeeinrichtung angeordnet ist, in die mindestens ein Lösemittel und /oder Reagenz eingebracht ist.

Außerdem ist eine erfindungsgemäße Vorrichtung bevorzugt, bei der auf das Absorptionsfeld und/oder die mindestens eine flüssigkeitführende Struktur mindestens ein Lösemittel und/ oder Reagenz direkt von außen und/oder durch einen auf das mindestens eine Speicherreservoir wirkenden Stimulus aufbringbar ist.

Die Aufgabe der vorliegenden Erfindung wird weiterhin gelöst durch ein Verfahren zur Identifizierung von Mikroorganismen unter Verwendung der erfindungsgemäßen Vorrichtung.

Die Aufgabe der Erfindung wird durch ein Verfahren zur Identifizierung von Mikroorganismen unter Verwendung der erfindungsgemäßen Vorrichtung gelöst, wobei man
a) einen Mikroporenfilter mit einer Probe beaufschlagt,
b) den Mikroporenfilter in einer Aussparung, auf einer Aussparung oder unter einer Aussparung einer Zwischenschicht anordnet,
c) mindestens ein Lösemittel und/oder Reagenz in mindestens eine Aufnahmevorrichtung des mindestens einen Absorptionsfeldes und/oder in mindestens eine flüssigkeitführende Struktur und/oder in mindestens ein Speicherreservoir einbringt,
d) das Speicherreservoir aus c) auf einem Absorptionsfeld anordnet,
e) die Bestandteile der Vorrichtung gemäß Anspruch 1 zusammensetzt,
f) mindestens einen Stimulus an mindestens ein Speicherreservoir anlegt oder direkt ein Lösemittel und/oder Reagenz von außen auf das saugfähige Material des Reaktionsfließes aufträgt, und
g) die in der Probe aus a) enthaltenden Mikroorganismen identifiziert.

Bevorzugt ist ein erfindungsgemäßes Verfahren, wobei mindestens ein Reagenz in c) in einer getrockneten Form vorliegt.

Besonders bevorzugt ist ein erfindungsgemäßes Verfahren, wobei mindestens ein Lösemittel oder Reagenz in d) in einer verfestigten Form vorliegt.

Insbesondere bevorzugt ist ein erfindungsgemäßes Verfahren, wobei der Stimulus in f) thermische Strahlung, eine Änderung im pH-Wert, UV-Strahlung oder ein elektrischer Stimulus ist.

Außerdem ist ein erfindungsgemäßes Verfahren bevorzugt, wobei der Stimulus die Verflüssigung des Lösemittels oder Reagenzes, welches auf und/oder in dem Absorptionsfeld angeordnet ist, auslöst.

Weiterhin ist ein erfindungsgemäßes Verfahren bevorzugt, wobei in g) die Mikroorganismen auf Grund eines Farbumschlages identifizierbar sind.

Im Zusammenhang mit der vorliegenden Erfindung können Lösemittel und/oder Reagenzien ausgewählt sein aus für Mikroorganismen spezifische Antikörper, die mit einem Enzym markiert sind, Enzyme, Redoxfarbstoffe, Wasser, Alkohole, verdünnte Säuren, Basen, Protonendonatoren oder -akzeptoren.

Des Weiteren können im Zusammenhang mit der vorliegenden Erfindung flüssigkeitführende Strukturen ausgewählt sein aus Kanälen, Röhren oder benetzbaren Fasernetzwerken, die bevorzugt eine Geometrie in der Größenordnung 10⁻³ bis 10⁻⁶ m aufweisen.

Außerdem sind im Zusammenhang mit der vorliegenden Erfindung Speicherreservoire Hydrogele oder Polymergele, die Wasser oder verdünnte Säuren speichern können und durch eine externe Energieänderung, bevorzugt durch Licht-, pH-Wert oder Temperaturstimulus wieder freisetzen können.

Weiterhin ist im Zusammenhang mit der vorliegenden Erfindung flüssigkeitsundurchlässiges Material ausgewählt aus doppelseitiger Klebefolie, Latex, Silikon, Parafin, einer Leimschicht oder einer Lackschicht.

Die vorliegende Erfindung wird mit den beigefügten Zeichnungen näher erläutert. Es zeigt:
Fig. 1 eine schematische Darstellung eines Aufbaus einer ersten Ausführungsform der erfindungsgemäßen Vorrichtung,
Fig. 2 eine schematische Darstellung einer zweiten Ausführungsform der erfindungsgemäßen Vorrichtung, und
Fig. 3 eine schematische Darstellung einer dritten Ausführungsform der erfindungsgemäßen Vorrichtung.

Die bekannten Testmethoden zur Identifizierung von Mikroorganismen nehmen bislang mehrere Tage bis Wochen in Anspruch, so dass entsprechende Anlagen mit Befund noch in der Testzeit in Betrieb sind, oder vorher positiv getestete Anlagen für den Analysezeitraum noch nicht freigegeben sind, obwohl keine Keimbelastung mehr vorliegt.

Die vorliegende Erfindung offenbart eine Vorrichtung und ein Verfahren mittels derer dieser Testzeitraum zur Identifizierung von Mikroorganismen auf die Dauer eines Schnelltestsystems mit wenigen Minuten verkürzt werden kann. Dabei ist es vorteilhaft, dass dieser Ablauf bei mindestens gleicher Sensitivität der bisher etablierten und gesetzlich akzeptierten Analysemethoden stattfindet.

Weiterhin vorteilhaft ist, dass die zu untersuchende Menge an Medium oder Gas in dem sich die zu identifizierenden Mikroorgansimen befinden frei variiert werden kann, ohne die Vorrichtung und/oder das Verfahren verändern zu müssen.

Ein weiterer Vorteil der erfindungsgemäßen Vorrichtung ist die Verwendung von Papier, cellulosebasierenden Substraten, Polyethylen, Polyethersulfon oder Polycarbonat als saugfähiges Material. Die Verwendung günstiger Materialien wirkt sich positiv auf die Herstellungskosten der erfindungsgemäßen Vorrichtung aus. Daher ist die erfindungsgemäße Vorrichtung auch als Einwegschnelltestsystem konzipiert.

Auf Grund der dicht miteinander verschließbaren Bestandteile der erfindungsgemäßen Vorrichtung, ist die Vorrichtung bei einem positiven Mikroorganismenbefund von ihrer Umwelt isoliert, wodurch eine Gefährdung von Mensch und Umwelt durch das Testsystem faktisch ausgeschlossen werden kann.

Erfindungsgemäß kann zur Bestimmung der Mikroorganismen ein spezifischer Antikörpernachweis verwendet werden. Vorteilhaft daran ist, dass dadurch der Gegenstand der vorliegenden Erfindung schnell auf eine beliebige Anzahl von zu identifizierenden Mikroorganismen angepasst werden kann, sofern ein hinreichend spezifischer Antikörper vorliegt.

Die nachfolgenden Beispiele erläutern unter Bezugnahme auf die Figuren die Erfindung näher, ohne den Umfang der Erfindung zu beschränken.

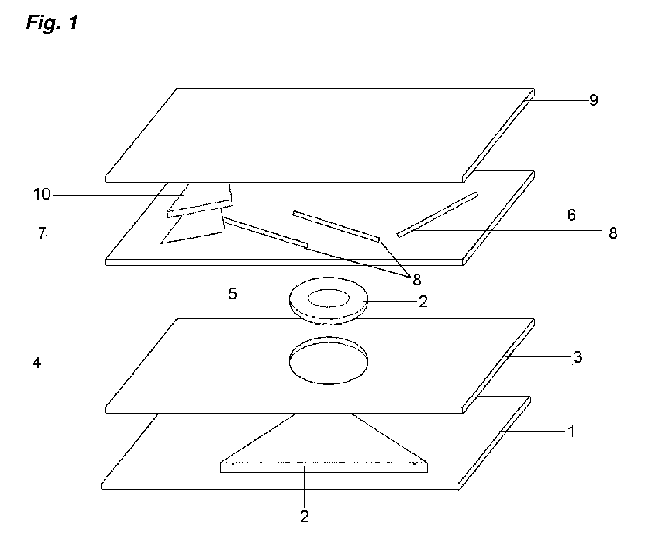

In Figur 1 wird eine erste bevorzugte Ausführungsform der erfindungsgemäßen Vorrichtung zur Identifizierung von Mikroorganismen dargestellt. Die erfindungsgemäße Vorrichtung umfasst einen Träger 1, auf den ein saugfähiges Material 2, beispielsweise Papier, fixiert ist. Das saugfähige Material 2 auf dem Träger 1 kann als Endreservoir fungieren, welches durchlaufende Flüssigkeiten auffängt. Auf dem saugfähigen Material 2 wird eine Zwischenschicht 3 angeordnet, die aus einem flüssigkeitsundurchlässigen Material besteht. Innerhalb der Zwischenschicht 3 ist eine Aussparung 4 eingelassen, in die ein Mikroporenfilter 5 mit dem zu identifizierenden Mikroorganismen angeordnet ist. Erfindungsgemäß kann der Mikroporenfilter 5 auf ein saugfähiges Material 2 gelegt werden. Das saugfähige Material 2 dient beispielsweise als Brücke, um den Höhenunterschied zwischen dem saugfähigen Material 2 auf dem Träger 1 und der Oberfläche der Zwischenschicht 3 auszugleichen. Der Mikroporenfilter 5 kann aber auch direkt innerhalb der Aussparung 4 auf das saugfähige Material 2, das auf dem Träger 1 fixiert ist, angeordnet werden.

Über der Zwischenschicht 3 ist ein Reaktionsfließ 6 angeordnet, das aus einem saugfähigen Material 2 besteht. In dem Reaktionsfließ 6 sind ein Absorptionsfeld 7 und drei flüssigkeitführende Strukturen 8, als Kanäle ausgebildet, angeordnet. Erfindungsgemäß können aber auch nur Absorptionsfelder 7 oder nur flüssigkeitführende Strukturen 8 in dem Reaktionsfließ 6 angeordnet sein, wobei die jeweilige Anzahl variabel ist. Die einzelnen Kanäle stellen dabei mit den Zwischenschichten und den Reaktionsfließen ein über mehrere Lagen verbundenes Kanalsystem dar.

Auf dem Absorptionsfeld 7 ist ein Speicherreservoir 10 angeordnet, wobei das Speicherreservoir 10 erfindungsgemäß ein Hydrogel oder ein Polymergel sein kann. Weiterhin sind in den flüssigkeitführenden Strukturen 8 Aufnahmevorrichtungen vorgesehen, die erfindungsgemäß flüssige oder getrocknete Reagenzien und/oder Lösemittel aufnehmen können. Außerdem können auch in oder auf die Absorptionsfelder 7 und in die Speicherreservoire 10 Lösemittel und/oder Reagenzien eingebracht werden.

Über dem Reaktionsfließ 6 wird eine Deckplatte 9 angeordnet. Die erfindungsgemäße Vorrichtung stellt zusammengebaut ein luftdicht verschlossenes, von der Umwelt isoliertes System dar. Dadurch wird bei einem positiven Befund eine Gefährdung von Mensch und Umwelt vermieden. Des Weiteren vorteilhaft ist die erfindungsgemäße Verwendung kostengünstiger Materialen, wodurch die Herstellungskosten reduziert werden. Zudem stellt die erfindungsgemäße Vorrichtung ein Einmalschnelltestsystem dar, das nach Verwendung verworfen wird. Daher müssen keine Wartezeiten, nach einem positiven Befund innerhalb eines Systems, bis zur erneuten Nutzung des Systems abgewartet werden.

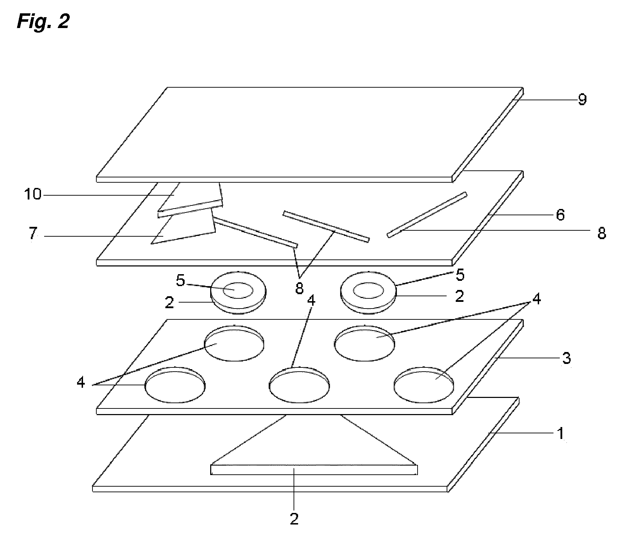

In Figur 2 wird eine zweite Ausführungsform der erfindungsgemäßen Vorrichtung gezeigt. In dieser ebenfalls bevorzugten Ausführungsform besteht der Unterschied zur Ausführungsform in Figur 1 darin, dass innerhalb der Zwischenschicht 3 mehrere Aussparungen 4 angeordnet sind. Erfindungsgemäß können dadurch eine Vielzahl verschiedener Mikroorganismen auf unterschiedlichen Mikroporenfiltern 5 gleicher oder verschiedener Proben identifiziert werden. Vorteilhaft daran ist der gleichzeitige Ablauf der Identifizierung von Mikroorganismen verschiedener Proben, was zu einer enormen Reduzierung des Zeitaufwands führt. Außerdem wird nur eine erfindungsgemäße Vorrichtung mit den entsprechend benötigten Materialien verwendet, was sich positiv auf die Herstellungskosten auswirkt.

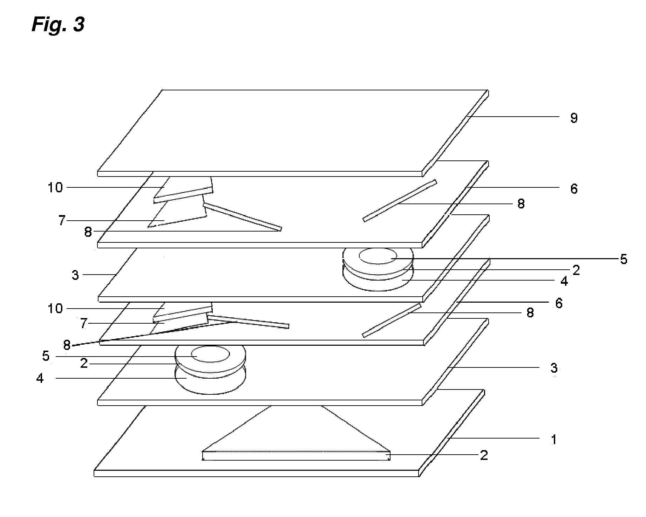

Eine dritte ebenfalls bevorzugte Ausführungsform der erfindungsgemäßen Vorrichtung ist in Figur 3 gezeigt. In dieser Ausführungsform umfasst die erfindungsgemäße Vorrichtung verglichen mit der Ausführungsform in Figur 1 eine zweite Zwischenschicht 3 und ein zweites Reaktionsfließ 6. Innerhalb der erfindungsgemäßen Vorrichtung sind die Zwischenschicht 3 und das Reaktionsfließ 6 abwechselnd angeordnet, wobei die Anzahl der sich insgesamt abwechselnden Lagen variabel ist. Dadurch können vorteilhafterweise innerhalb einer erfindungsgemäßen Vorrichtung mehrere Analysereaktionen einer Probe parallel zueinander ablaufen. Beispielsweise kann die eine Reaktion eine Nachweisreaktion zur Identifizierung der Mikroorganismen auf einem Mikroporenfilter 5 darstellen und die andere Reaktion ein Nachweis dafür sein, ob die zu identifizierenden Mikroorganismen auf einem zweiten Mikroporenfilter 5 noch leben.

Die nachfolgenden Ausführungsbeispiele beschreiben das erfindungsgemäße Verfahren unter Verwendung der erfindungsgemäßen Vorrichtung näher, ohne den Umfang der Erfindung zu beschränken.

### Beispiel 1

Aufgrund der notwendigen Sicherheitsbeschränkungen S2 für pathogene Mikroorganismen (hier *Legionellen*) wurde die erfindungsgemäße Vorrichtung und das erfindungsgemäße Verfahren mit *E. coli* getestet. Durch die Verwendung eines spezifischen Antikörpers gegen einen alternativen Mikroorganismus, beispielsweise *Legionella spec.*, *Salmonella spec.* ist das Testsystem jedoch auf jeden beliebigen anderen Mikroorganismus erweiterbar.

Zunächst wird die erfindungsgemäße Vorrichtung vorbereitet. Dafür wird ein spezifischer Antikörper, der mit einer Peroxidase markiert ist in eine erste Aufnahmevorrichtung in einem linearen Kanal innerhalb des Reaktionsfließes trocken vorgelagert. Als saugfähiges Material, aus dem das Reaktionsfließ besteht, wird beispielsweise ein kanalstrukturiertes Papier verwendet. In einer zweiten Aufnahmevorrichtung des gegabelten Kanals wird ein Redoxfarbstoff, beispielsweise Tetramethylbenzidin, im getrockneten Zustand vorgelagert. Im gleichen Kanal wird in einer dritten Aufnahmevorrichtung ein Peroxid als Protonendonator trocken vorgelagert.

Anschließend wird auf ein erstes Absorptionsfeld ein Speicherreservoir in Form eines wasserspeichernden Hydrogels aufgebracht. Auf ein zweites Absorptionsfeld wird ein Hydrogel aufgebracht, in dem eine verdünnte Schwefelsäure in gespeicherter Form vorliegt.

Mit der Deckplatte wird die erfindungsgemäße Vorrichtung von oben dicht verschlossen. Auf den Träger wird mit Hilfe einer Zwischenschicht, bestehend aus doppelseitiger Klebefolie ein Endreservoir aus einem saugfähigen Material fixiert. In der Mitte der doppelseitigen Klebefolie wird eine Aussparung eingebracht, welche mit einer Verbindungsbrücke aus saugfähigem Material ausgefüllt ist. Die so vorbereitete erfindungsgemäße Vorrichtung ist nun einsatzbereit.

Ein Mikroporenfilter wird mit Hilfe einer gängigen Haltevorrichtung mit dem zu überprüfenden gas- oder flüssigkeitführendem System verbunden. Anschließend wird eine vorher fest definierte Zielvolumenmenge an Medium durch den Mikroporenfilter geleitet.

Danach wird die Halterung vom zu testenden System gelöst und der Mikroporenfilter entnommen. Die dem Fluss entgegen gerichtete Seite des Mikroporenfilters wird als Oberseite definiert. Die Filterseite in Flussrichtung wird als Unterseite definiert.

Der entnommene Mikroporenfilter wird nun mit der Unterseite mittig auf die Verbindungsbrücke, die aus saugfähigem Material besteht, innerhalb der Aussparung der Zwischenschicht geklebt. Danach wird der Träger mit der Deckplatte zentriert, handfest verpresst und somit die erfindungsgemäße Vorrichtung luftdicht verschlossen.

Über einen thermischen Stimulus, hier beispielsweise 38°C, wird das Hydrogel auf dem ersten Absorptionsfeld zum Kollabieren gebracht, wodurch das darin gespeicherte Wasser freigesetzt wird. Die Kapillarkräfte des saugfähigen Materials ziehen das Wasser in die erste Aufnahmevorrichtung des linearen Kanals, wo es den dort zuvor gelagerten Antikörper auflöst. Im Weiteren wird das Wasser durch den Mikroporenfilter hindurch, bis hin zum Endreservoir gezogen. Im Mikroporenfilter wird ein Teil der Antikörper an den dort vorhandenen zu identifizierenden Mikroorganismen spezifisch gebunden.

In einem zweiten Schritt wird das Hydrogel, das auf dem zweiten Absorptionsfeld angeordnet ist, ebenfalls bei 38°C thermisch zum Kollaps gebracht. Dadurch wird die verdünnte Schwefelsäure freigesetzt. Diese wird ebenfalls durch die Kapillarkräfte des saugfähigen Materials in den Kanal gezogen, wo sie an einer dritten Aufnahmeeinrichtung das Peroxid auflöst und mit diesem reagiert. Das Reaktionsprodukt wird weiter zur zweiten Aufnahmevorrichtung gezogen und durchfeuchtet dort das Tetramethylbenzidin besetzte saugfähige Material.

Hierbei kommt es zum Kontakt zwischen dem bereits vorher durchfeuchteten Mikroporenfilter und dem Tetramethylbenzidin. Die zuvor mit dem spezifischen Antikörper markierten Mikroorganismen setzen mit Hilfe der an den Antikörper gebundenen Peroxidase den Redoxfarbstoff um. Auf Grund der Umsetzung wird ein Farbumschlag sichtbar, im vorliegenden Ausführungsbeispiel von gelbgrün nach blau.

Anhand der Geschwindigkeit des Farbumschlags kann der Nutzer der vorliegenden Erfindung den positiven Befund der zu identifizierenden Mikroorganismen ablesen.

Alternativ kann das erfindungsgemäße Verfahren auch automatisiert photometrisch und/oder mit Hilfe von zuvor in das System eingebrachten Elektroden aus leitfähigen Materialien ablaufen. Dabei wird der Befund elektrochemisch ausgelesen und somit anschließend direkt elektronisch ausgewertet.

Vorteilhaft an der vorliegenden Erfindung ist die Kombination von Mikroporenfiltern mit definierter Porengröße und der Aufkonzentration der zu identifizierenden Mikroorganismen mit einem beispielsweise papierbasierten Mikrofluidikschnelltestsystem mit auf einem Redoxfarbstoff basiertem Antikörpernachweis. Weiterhin positiv ist die Verwendung responsiver Hydrogele, wodurch die vorliegende Erfindung vollkommen automatisiert durchgeführt werden kann und daher frei von Anwenderfehlern ist. Außerdem ist an der vorliegenden Erfindung vorteilhaft, dass diese bei Bedarf auch ohne die Verwendung von Hydrogelen oder Polymergelen betrieben werden kann, wobei die Lösungen dann von außen dem System extern zugeführt werden können.

### Beispiel 2

In einem zweiten Ausführungsbeispiel der vorliegenden Erfindung wird die erfindungsgemäße Vorrichtung und das erfindungsgemäße Verfahren grundsätzlich, wie bereits in Beispiel 1 beschrieben, aufgebaut und durchgeführt. Im Unterschied zum ersten Ausführungsbeispiel werden bei der Vorbereitung der erfindungsgemäßen Vorrichtung jedoch zwei Zwischenschichten und zwei Reaktionsfließe abwechselnd nacheinander zwischen dem Träger und der Deckplatte angeordnet.

Erfindungsgemäß dient ein derartiger Aufbau dazu, zum Einen die vorhandenen Mikroorganismen auf einem Mikroporenfilter zu identifizieren und zum Anderen, um zu analysieren, ob die Mikroorganismen in der zu untersuchenden Probe noch leben. Dieses lässt sich durch einen Farbnachweis von Stoffwechselprodukten, beispielsweise Adenosintriphosphat (ATP) bewerkstelligen.

Zunächst wird das erste Reaktionsfließ entsprechend, wie bereits in Beispiel 1 beschrieben, vorbereitet und unter das Reaktionsfließ wird eine Zwischenschicht mit der Aussparung für einen Mikroporenfilter angeordnet.

Danach wird ein zweites Reaktionsfließ ähnlich zum ersten Reaktionsfließ vorbereitet, wobei dabei verschiedene Reagenzien und/oder Lösemittel in Hydrogelen, auf den Absorptionsfeldern und oder in den entsprechenden Aufnahmevorrichtungen der flüssigkeitführenden Strukturen in einem flüssigen, verfestigten und/oder getrockneten Zustand aufgebracht werden. Dieser Aufbau kann dazu verwendet werden, um einen Lebensnachweis der Mikroorganismen durchzuführen. Die hierfür benötigten Reagenzien und/oder Lösemittel sind dem Fachmann bekannt.

Anschließend findet der gleiche Versuchsablauf, wie in Beispiel 1 beschrieben statt, wobei zwei Mikroporenfilter verwendet werden, um die Proben aus einem gas- oder flüssigkeitführenden System zu übernehmen.

Danach wird der eine Mikroporenfilter in einer Aussparung der ersten Zwischenschicht angeordnet und der andere Mikroporenfilter in einer Aussparungen der zweiten Zwischenschicht.

Vorteilhaft an diesem Ausführungsbeispiel ist, dass die unterschiedlichen Analysen der Mikroorganismen, die Identifizierung und der Lebensnachweis in der gleichen erfindungsgemäßen Vorrichtung ablaufen können. Dies wirkt sich positiv auf die Herstellungskosten und den Zeitaufwand aus. Dabei ist es außerdem vorteilhaft, dass die Flüssigkeitsvolumina, beispielsweise in den Hydrogelen, individuell einstellbar sind. Daher ist es möglich das Volumen derart einzustellen, dass ein Durchtränken des zweiten Reaktionsfließes mit der Flüssigkeit aus dem ersten Reaktionsfließ vermieden wird. Außerdem vorteilhaft an der erfindungsgemäßen Ausführungsform ist, dass selbst ein Durchtränken des zweiten Reaktionsfließes nicht zu einer falschen Aussage des zweiten Reaktionsnachweises führen würde, da unterschiedliche Reagenzien und/oder Lösemittel im ersten und zweiten Reaktionsfließ verwendet werden.

### Bezugszeichenliste

- 1: Träger
- 2: Saugfähiges Material
- 3: Abdichtende Zwischenschicht
- 4: Aussparung
- 5: Mikroporenfilter
- 6: Reaktionsfließ
- 7: Absorptionsfeld
- 8: Flüssigkeitführende Struktur
- 9: Deckplatte
- 10: Speicherreservoir

## Patentansprüche

1. Vorrichtung zur Identifizierung von Mikroorganismen umfassend:
a) einen Träger (1), wobei auf dem Träger (1) ein saugfähiges Material (2) angeordnet ist,
b) mindestens eine abdichtende Zwischenschicht (3), welche mindestens eine Aussparung (4) aufweist, welche zur Aufnahme zu identifizierender Mikroorganismen, die auf einem Mikroporenfilter (5) vorhanden sind, ausgestaltet ist,
c) mindestens ein Reaktionsfließ (6) bestehend aus einem saugfähigen Material (2), auf dem mindestens ein Absorptionsfeld (7) und/oder mindestens eine flüssigkeitführende Struktur (8) zur Aufnahme von Reagenzien zur Identifizierung der Mikroorganismen angeordnet ist, und
d) eine Deckplatte (9).

2. Vorrichtung, gemäß Anspruch 1, **dadurch gekennzeichnet, dass** der Träger (1) und/oder die Deckplatte (9) aus Glas, Polyethylen, Polypropylen, Polyvinylchlorid, Polystyrol, Cyclo-Olefin-Copolymere, Polymethylmethacrylat oder Polyurethan besteht.

3. Vorrichtung, gemäß Anspruch 1, **dadurch gekennzeichnet, dass** das saugfähige Material (2) aus Papier, cellulosebasierenden Substraten, Polyethylen, Polyethersulfon oder Polycarbonat besteht.

4. Vorrichtung, gemäß Anspruch 1, **dadurch gekennzeichnet, dass** die abdichtende Zwischenschicht (3) aus einem flüssigkeitsundurchlässigem Material besteht.

5. Vorrichtung, gemäß Anspruch 4, **dadurch gekennzeichnet, dass** die abdichtende Zwischenschicht (3) fest mit dem saugfähigen Material (2) verklebt oder verpresst ist.

6. Vorrichtung, gemäß Anspruch 1, **dadurch gekennzeichnet, dass** mehr als ein Reaktionsfließ (6) und mehr als eine abdichtende Zwischenschicht (3) vorgesehen ist, wobei das Reaktionsfließ (6) und die abdichtende Zwischenschicht (3) abwechselnd angeordnet sind.

7. Vorrichtung, gemäß Anspruch 1, **dadurch gekennzeichnet, dass** auf dem mindestens einen Absorptionsfeld (7) mindestens ein schaltbares Speicherreservoir (10) angeordnet ist.

8. Vorrichtung gemäß Anspruch 7, **dadurch gekennzeichnet, dass** das mindestens eine Speicherreservoir (10) mit einer flüssigkeitführenden Struktur (8) in Verbindung steht.

9. Vorrichtung, gemäß Anspruch 7 oder 8, **dadurch gekennzeichnet, dass** das schaltbare Speicherreservoir (10) ein Polymergel ist.

10. Vorrichtung, gemäß mindestens einem der Ansprüche 7 bis 9, **dadurch gekennzeichnet, dass** innerhalb des Speicherreservoirs (10) ein Lösemittel und/oder Reagenz eingebracht ist.

11. Vorrichtung, gemäß Anspruch 1, **dadurch gekennzeichnet, dass** innerhalb des mindestens einen Absorptionsfeldes (7) und/oder der mindestens einen flüssigkeitführenden Struktur (8) mindestens eine Aufnahmeeinrichtung angeordnet ist, in die mindestens ein Lösemittel und/oder Reagenz eingebracht ist.

12. Vorrichtung, gemäß mindestens einem der Ansprüche 1 bis 11, **dadurch gekennzeichnet, dass** auf das Absorptionsfeld (7) und/oder die mindestens eine flüssigkeitführende Struktur (8) mindestens ein Lösemittel und/ oder Reagenz direkt von außen und/oder durch einen auf das mindestens eine Speicherreservoir (10) wirkenden Stimulus aufbringbar ist.

13. Verfahren zur Identifizierung von Mikroorganismen mittels einer Vorrichtung gemäß der Ansprüche 1 bis 12, wobei man
a) einen Mikroporenfilter (5) mit einer Probe beaufschlagt,
b) den Mikroporenfilter (5) in einer Aussparung (4), auf einer Aussparung (4) oder unter einer Aussparung (4) einer Zwischenschicht (3) anordnet,
c) mindestens ein Lösemittel und/oder Reagenz in mindestens eine Aufnahmevorrichtung des mindestens einen Absorptionsfeldes (7) und/oder in mindestens eine flüssigkeitführende Struktur (8) und/oder in mindestens ein Speicherreservoir (10) einbringt,
d) das Speicherreservoir (10) aus c) auf einem Absorptionsfeld (7) anordnet,
e) die Bestandteile der Vorrichtung gemäß Anspruch 1 zusammensetzt,
f) mindestens einen Stimulus an mindestens ein Speicherreservoir (10) anlegt oder direkt ein Lösemittel und/oder Reagenz von außen auf das saugfähige Material (2) des Reaktionsfließes (7) aufträgt, und
g) die in der Probe aus a) enthaltenden Mikroorganismen identifiziert.

14. Verfahren, gemäß Anspruch 13, **dadurch gekennzeichnet, dass** mindestens ein Reagenz in c) in einer getrockneten Form vorliegt.

15. Verfahren, gemäß Anspruch 13, **dadurch gekennzeichnet, dass** mindestens ein Lösemittel oder Reagenz in d) in einer verfestigten Form vorliegt.

16. Verfahren, gemäß Anspruch 13, **dadurch gekennzeichnet, dass** der Stimulus in f) thermische Strahlung, UV-Strahlung, eine Änderung im pH-Wert oder ein elektrischer Stimulus ist.

17. Verfahren, gemäß Anspruch 16, **dadurch gekennzeichnet, dass** der Stimulus die Verflüssigung des Lösemittels oder Reagenzes, welches auf und/oder in dem Absorptionsfeld (7) angeordnet ist, auslöst.

18. Verfahren, gemäß Anspruch 13, **dadurch gekennzeichnet, dass** in g) die Mikroorganismen auf Grund eines Farbumschlages identifizierbar sind.
